# EUROPÄISCHE PATENTANMELDUNG

(11) **EP 1 156 322 A2**
(43) Veröffentlichungstag der Anmeldung: **21.11.2001**
(21) Anmeldenummer: 01110315.7
(22) Anmeldetag: 26.04.2001
(51) Int. Cl.: G01N 27/04

(54) **Feuchtemessanordnung**

(30) Priorität: 26.04.2000 DE 10020455; 25.05.2000 DE 10025902
(71) Anmelder: Mahlo GmbH & Co. KG, 93342 Saal (DE)
(72) Erfinder: Klaehsen, Helmut, 47608 Geldern (DE)
(74) Vertreter: Bohnenberger, Johannes, Dr.

(57) **Zusammenfassung**

Feuchtemeßanordnung (1) zur Messung des Feuchtegehaltes in einer bewegten Bahn flächigen Materials, insbesondere einer Textilbahn (7), wobei eine insbesondere durch die Materialbahn angetriebene Meßwalze vorgesehen ist, bei der die Meßwalze (3) eine segmentierte Oberfläche mit mindestens zwei durch hochohmige Isolationsabschnitte (3.3, 3.4) voneinander getrennten leitfähigen Umfangsbereichen (3.1, 3.2) aufweist, welche jeweils mit einem elektrischen Anschluß zur Zuführung bzw. zum Abgriff eines Meßstromes verbunden sind.

## Beschreibung

Die Erfindung betrifft eine Feuchtemeßanordnung nach dem Oberbegriff des Anspruchs 1. Eine solche Anordnung ist insbesondere zur Bestimmung des Restfeuchtegehaltes von Textilien, Papier oder speziellen Kunststofffolien, beispielsweise fotografischen Filmen, bestimmt.

Die Bestimmung der Restfeuchte von Textilien, Papier o. ä. flächigem Material unter Nutzung ihrer Leitfähigkeit - auch bezeichnet als "konduktometrische Messung" - ist seit langem bekannt. Hierbei wird die logarithmische Abhängigkeit des elektrischen Widerstandes des flächigen Materials von seinem Feuchtegehalt, der weitgehend unabhängig von der Flächenmasse ist, genutzt. Es ist auch bekannt, als Meßelektroden hierfür Walzen oder Rollen zu nutzen, wobei der elektrische Widerstand zwischen den Walzen bzw. Rollen eines Paares gemessen wird. Es kann sich hierbei um zwei Meßelektroden oder eine Meßelektrode und eine Führungswalze der Bahn handeln.

Weiterhin ist es - etwa aus der DD-A-97 751 - bekannt, die elektrostatische Aufladung zur Bestimmung des Feuchtegehaltes einer Flächenbahn zu nutzen.

Aus der DE-A-43 21 322 ist eine Feuchtemessung im unteren Restfeuchtebereich unter Einsatz einer über der Flächenbahn angeordneten Hochspannungs-Elektrode bekannt. Über diese Elektrode wird in der Bahn eine Ladungsspur influenziert, deren Ladungsdichte als Funktion des elektrischen Widerstandes (und somit des Feuchtegehaltes) mittels nachfolgend angeordneter Sonden berührungslos gemessen wird.

Aus der DE-A-42 32 185 ist ein weiteres berührungsloses Meßverfahren bekannt, bei dem in einem lokalen Bereich der bewegten Flächenbahn ein elektrisches Feld erzeugt und die durch den feuchteabhängigen Gleichstromwiderstand der Bahn auf dieses Feld verursachte abschirmende Wirkung gemessen wird.

Die bekannten elektrischen Meßverfahren und -anordnungen zur Messung des Feuchtegehaltes von bewegten flächigen Materialbahnen haben sich mehr oder weniger gut bewährt, aber auch bestimmte Nachteile gezeigt. Speziell bei den gattungsgemäßen konduktometrischen Feuchtemeßanordnungen mit mehreren Meßwalzen haben sich Probleme durch unerwünschte Einwirkungen auf empfindliches Material (Streifigkeit) ergeben, und zudem sind diese Meßanordnungen im mechanischen und elektrischen Aufbau vergleichsweise aufwendig.

Der Erfindung liegt daher die Aufgabe zugrunde, eine verbesserte Feuchtemeßanordnung der gattungsgemäßen Art anzugeben, die insbesondere mit geringem Konstruktionsaufwand auskommt und grundsätzlich zur Messung sehr unterschiedlicher - auch besonders niedriger - Restfeuchtegehalte ohne nachteilige Einflüsse auf empfindliches flächiges Material geeignet ist.

Diese Aufgabe wird durch eine Feuchtemeßanordnung mit den Merkmalen des Anspruchs 1 gelöst.

Die Erfindung schließt den wesentlichen Gedanken ein, zur Feuchtemessung eine Walze mit einem Umfangsbereich vorzusehen, der voneinander isolierte leitfähige Bereiche aufweist. Steht eine solche Walze mit zwei durch einen isolierenden Bereich voneinander getrennten leitfähigen Abschnitten mit einem flächigen Material in Kontakt, so wird sich zwischen den leitfähigen Bereichen über das flächige Material ein Strompfad ausbilden, wenn an den leitfähigen Bereichen Stromanschlüsse vorgesehen sind und wenn der Isolationswiderstand des in der Walze vorgesehenen isolierenden Bereiches wesentlich größer als derjenige des flächigen Materials selbst ist. Aus der gemessenen Stromstärke und der daraus erhaltenen Leitfähigkeit kann dann in an sich bekannter Weise auf den Feuchtegehalt des flächigen Materials geschlossen werden.

In einer wegen ihrer Einfachheit und der besonders schonenden Behandlung des Meßgutes bevorzugten Ausführung wird eine einzelne Meßwalze dieser Art eingesetzt, die eine mindestens der Breite der Materialbahn entsprechende Länge hat.

In einer weiteren besonders einfachen Ausführung weist die Meßwalze ein Paar von zylindersegmentförmigen leitfähigen Umfangsbereichen auf, die einen einzelnen Feuchtemeßabschnitt bestimmten. Die Begrenzungskanten der leitfähigen Abschnitte verlaufen hierbei parallel zur Drehachse der Walze.

In einer hierzu alternativen Ausführung sind die Begrenzungskanten der leitfähigen Abschnitte nicht-parallel zur Walzenachse; insbesondere haben sie einen dieser gegenüber geneigten bzw. "spiraligen" Verlauf. Bei der letztgenannten Ausführung steht bei einer auf der ebenen Materialbahn aufliegenden Meßwalze (über deren Breite gesehen) jeweils fortlaufend ein anderer kleiner Bereich des Isolationsabschnittes mit dem Meßgut in Kontakt. Es wird also sukzessive jeweils eine lokale Strom- und damit Feuchtigkeitsmessung über die Breite der Materialbahn hinweg ausgeführt.

In einer weiteren bevorzugten Ausführung, die eine kontinuierliche Strommessung und damit besonders einfache Auswertung des Meßsignals ermöglicht, ist eine Umschlingung der Meßwalze durch das flächige Material vorgesehen. Der Umschlingungswinkel ist in Abstimmung auf den Aufbau der Meßwalze zu wählen und liegt bei einer besonders einfach aus zwei annähernd halbzylindrischen leitfähigen Bereichen mit dazwischen liegenden dünnen Isolationsstegen aufgebauten Meßwalze bei etwa 180°. Hier existiert bei jeder Walzenstellung ein Strompfad zwischen den leitfähigen Abschnitten über die Materialbahn.

Dies gilt im übrigen auch für die vorgenannte Ausführung mit geneigten Begrenzungskanten der leitfähigen Abschnitte, wenn die Neigung geeignet gewählt ist - und zwar ohne Umschlingung der Meßwalze durch die Materialbahn.

Eine vielfältigen praktischen Anforderungen genügende Meßanordnung umfaßt eine Meßwalze mit drei Paaren leitfähiger Bereiche, die in Längsrichtung der Walze - jeweils durch Isolationsbereiche voneinander getrennt - aneinander gereiht sind. Hierbei hat der mittlere Bereich insbesondere eine Länge von ca. 500 mm, während die beiden Rand-Meßbereiche eine in Abhängigkeit von der Materialbreite gewählte Länge haben. Es versteht sich, daß bei dieser Meßanordnung drei Meßverstärker für die einzelnen Stromsignale vorgesehen sind. In einer modifizierten Ausführung können auch mehr als drei Meßsegment-Paare und somit mehr als drei Feuchtemeßabschnitte vorgesehen sein.

In einer weiteren, relativ leicht herstellbaren und gleichwohl vielfältigen Meßanforderungen genügenden, Ausführung umfaßt die Meßwalze ebenfalls drei Paare leitfähiger Bereiche, die über die Länge der Walze verteilt sind. Diese Ausführung unterscheidet sich von der vorgenannten dadurch, daß die leitfähigen Bereiche jeweils als geschlossene Ringe ausgeführt und zwischen ihnen schmale isolierende Ringe vorgesehen sind. Hierbei wird die Leitfähigkeit in einem Gewebsabschnitt, auf dem die Walze aufliegt, als Leitfähigkeit der Strompfade zwischen den jeweils zu einem Paar gruppierten leitfähigen Ringen gemessen. Auch diese Ausführung ist hinsichtlich der Anzahl der Meßring-Paare und deren geometrischer Abmessungen, insbesondere der Breite der isolierenden Ringe zwischen den leitfähigen Bereichen, frei auf die konkrete Meßaufgabe anpaßbar.

Die elektrischen Anschlüsse der leitfähigen Umfangsbereiche sind bevorzugt als Schleifringkontakte mit voneinander isolierten leitfähigen Ringen an mindestens einer der Stirnflächen der Meßwalze und jeweils einem zugeordneten Schleifkontakt, insbesondere einer Kohlebürste, ausgeführt. Alternativ hierzu können bei einer über die Breite der Materialbahn hinaus entsprechend verlängerten Meßwalze die Stromzuführungen und -abgriffe auch auf der Walzenumfangsfläche vorgesehen sein.

Insgesamt besteht ein enger Zusammenhang zwischen der geometrischen Ausführung der Oberfläche der Meßwalze und der Auswertungsschaltung für die Stromsignale sowie der Auslegung der Anordnung für bestimmte feuchte Bereiche des Meßgutes. Eine die Auswertung von Impulssignalen ermöglichende Auswertungsschaltung kann in vorteilhafter Weise mit einer Meßwalze und einer geometrischen Anordnung des flächigen Materials bezüglich der Meßwalze arbeiten, bei der ein kontinuierliches Stromsignal grundsätzlich nicht oder jedenfalls nicht unter allen Meßbedingungen geliefert wird. So ist mit einer entsprechenden - für den Fachmann grundsätzlich ohne weiteres realisierbaren - Stromimpuls-Auswertungsschaltung neben einer Anordnung zur Messung niedriger Restfeuchtegehalte, bei der die Breite bzw. Winkelerstreckung der Isolationsabschnitte wesentlich kleiner als diejenige der leitfähigen Abschnitte ist, gegebenenfalls zur Messung höherer Feuchtegehalte auch eine Meßanordnung ausführbar, bei der die Breite bzw. Winkelerstreckung der Isolationsabschnitte in der gleichen Größenordnung wie die Breite bzw. Winkelerstreckung der leitfähigen Abschnitte liegt.

Vorteile und Zweckmäßigkeiten der Erfindung ergeben sich im übrigen aus den Unteransprüchen sowie der nachfolgenden Beschreibung bevorzugter Ausführungsbeispiele anhand der Figuren. Von diesen zeigen:
- Fig. 1: eine perspektivische Prinzipskizze einer Feuchtemeßanordnung gemäß einer ersten Ausführungsform der Erfindung,
- Fig. 2: eine Prinzipskizze (in perspektivischer Darstellung) der Meßwalze einer Feuchtemeßanordnung gemäß einer zweiten Ausführungsform,
- Fig. 3: eine Prinzipskizze (in perspektivischer Darstellung) der Meßwalze einer Feuchtemeßanordnung gemäß einer dritten Ausführungsform,
- Fig. 4A und 4B: schematische Querschnittsdarstellungen von Meßwalzen gemäß weiteren Ausführungsformen und
- Fig. 5: eine Prinzipskizze (in perspektivischer Darstellung) der Meßwalze einer Feuchtemeßanordnung gemäß einer weiterem Ausführungsform der Erfindung.

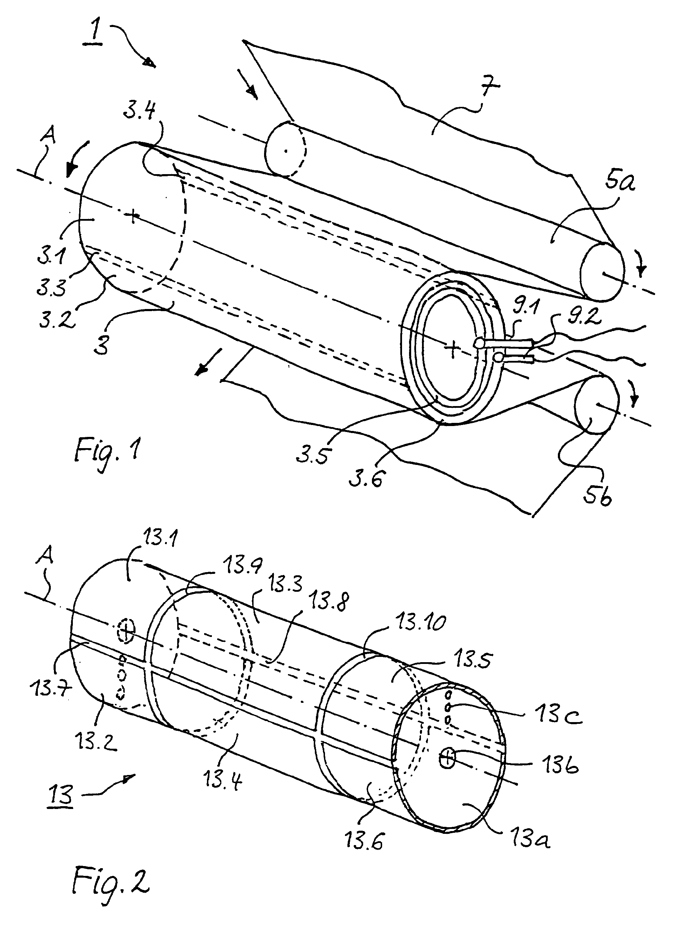

Fig. 1 zeigt eine Feuchtemeßanordnung 1 mit einer segmentierten Meßwalze 3, über die mit einem Umschlingungswinkel von etwas mehr als 180° mit Hilfe von zwei Hilfswalzen 5a, 5b eine Textilbahn 7 mit vorbestimmter Spannung geführt wird. Die Meßwalze 3 ist ohne eigenen Antrieb und wird durch die mit Spannung aufliegende Textilbahn 7 angetrieben.

Die Meßwalze 3 hat einen segmentierten Mantelbereich mit zwei annähernd halbzylindrischen leitfähigen Umfangsbereichen 3.1, 3.2 sowie zwei zwischen diesen liegenden schmalen, hochohmig isolierenden Stegen 3.3, 3.4. Auf der Stirnseite der Walze sind zwei konzentrische, gegeneinander isolierte Messingringe 3.5, 3.6 angebracht, die in der Walze mit den leitfähigen Umfangsbereichen 3.1 bzw. 3.2 verbunden sind. Den Messingringen 3.5, 3.6 sind je eine Kohlebürste 9.1, 9.2 zugeordnet, die die Meßwalze extern an einen (nicht gezeigten) Meßstromkreis anschließen.

Bei der gezeigten Anordnung ist in jeder Drehstellung der Meßwalze 3 einer der isolierenden Stege 3.3, 3.4 auf deren Umfangsfläche in Kontakt mit der Textilbahn 7, so daß diese permanent einen Teil des Meßstromkreises bildet und diesen schließt. Es fließt also während des Transports der Textilbahn ständig ein Meßstrom, aus dem mittels einer (an sich bekannten) Meßanordnung die Leitfähigkeit und damit der Feuchtgehalt bestimmt werden kann.

In Fig. 2 ist eine modifizierte Meßwalze 13 skizzenartig dargestellt, mit der über die Längserstreckung der Meßwalze - d. h. über die Breitenerstreckung der Textilbahn - drei voneinander getrennte Meßbereiche bestimmt werden. Die Meßwalze 13 umfaßt sechs leitfähige Umfangsbereiche 13.1 bis 13.6, die durch zwei isolierende Stege 13.7, 13.8 sowie zwei isolierende Ringe 13.9, 13.10 voneinander hochohmig isoliert sind. Im Walzengrundkörper 13a sind eine zentrale Bohrung 13b zur Aufnahme einer (nicht dargestellten) Lagerachse sowie weitere axiale Bohrungen für Anschlußleitungen zur Verbindung der leitfähigen Umfangsbereiche mit stirnseitig aufgesetzten (hier nicht dargestellten) leitfähigen Ringen in Art der in Fig. 1 gezeigten Messingringe vorgesehen.

Die in Fig. 2 skizzierte Meßwalze 13 wird in einer Meßanordnung gemäß Fig. 1 anstelle der dortigen Meßwalze 3 eingesetzt und erlaubt die getrennte Erfassung der Restfeuchte der Textilbahn 7 im Mittenbereich bzw. den Randbereichen.

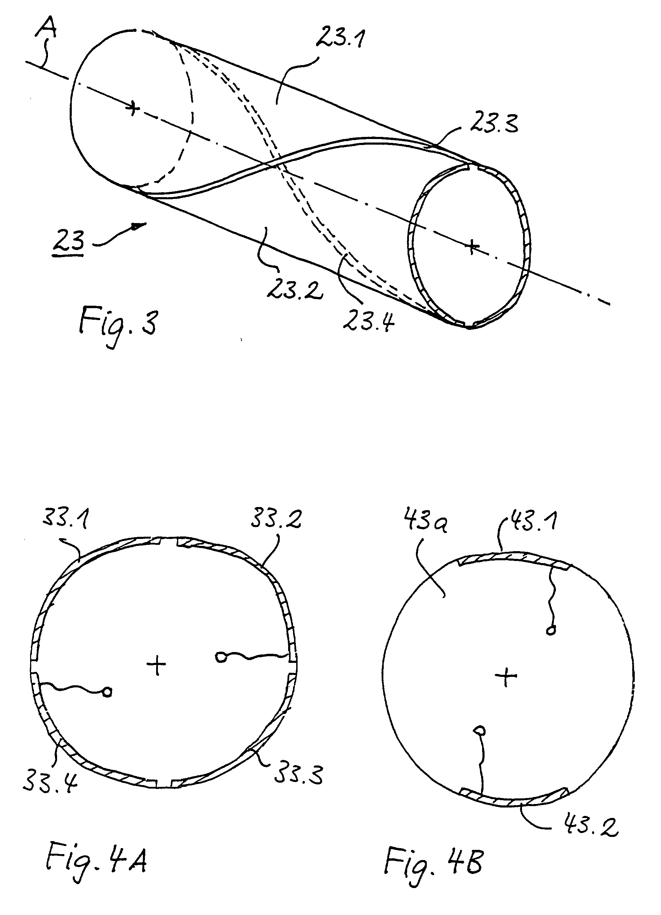

Fig. 3 zeigt die Prinzipskizze einer weiteren modifizierten Meßwalze 23, die sich von der Meßwalze 3 nach Fig. 1 dadurch unterscheidet, daß auf ihrem Umfang zwei leitfähige Umfangsbereiche 23.1, 23.2 mit gegenüber der Walzenachse A geneigten Längskanten vorgesehen sind. Auch hier sind zwischen den leitfähigen Umfangsbereichen hochohmig isolierende Stege 23.3, 23.4 vorgesehen. Durch die geneigte Ausführung der Längskanten der leitfähigen Umfangsbereiche 23.1, 23.2 ergibt sich insgesamt eine spiralige Segmentierung der Walzenoberfläche. Bei der in Fig. 3 gezeigten Ausführung mit zwei leitfähigen Bereichen, die jeweils knapp 180° des Walzenumfangs abdecken, und dazwischen liegenden schmalen isolierenden Bereichen läßt sich ein im wesentlichen kontinuierlich fließender Meßstrom ohne eine die Meßwalze umschlingende Führung der Textilbahn erreichen. Hierzu ist der Neigungswinkel auf die Länge der Meßwalze in der in der Figur gezeigten Art derart abzustimmen, daß bei jeder Winkelstellung der Meßwalze ein Punkt eines der isolierenden Stege 23.3, 23.4 mit einer ebenen Textilbahn in Kontakt steht, so daß sich dort ein über die Textilbahn verlaufender Strompfad zwischen den leitfähigen Umfangsabschnitten ausbilden kann.

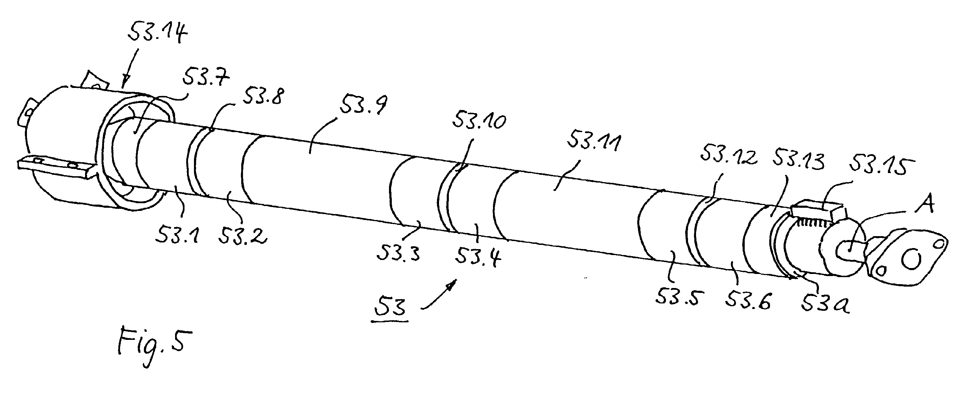

In Fig. 4A und 4B sind (lediglich beispielhaft) weitere Ausführungen der Meßwalzen-Segmentierung gezeigt.

Bei der in Fig. 4A gezeigten Variante mit vier über den Umfang verteilten leitfähigen Umfangsbereichen 33.1 bis 33.4 und jeweils dazwischen angeordneten (hier nicht gesondert bezeichneten) isolierenden Stegen kann in einer der Anordnung 1 nach Fig. 1 ähnlichen Meßanordnung mit einem kleineren Umschlingungswinkel der Meßwalze von ca. 90° gearbeitet werden. Die symbolisch dargestellten Anschlüsse an den leitfähigen Umfangsabschnitt 33.2, 33.4 verdeutlichen, daß die Meßstromzuführung bzw. -ableitung nur an diesen beiden Abschnitten erfolgt, während die dazwischen liegenden Umfangsabschnitte 33.1, 33.3 ohne Anschlüsse lediglich zur Schließung des Strompfades zwischen den mit Anschlüssen versehenen Bereichen dienen.

In Fig. 4B ist eine weitere Meßwalze 43 im Querschnitt skizziert, bei der zwei leitfähige Umfangsbereiche 43.1, 43.2 in einem isolierenden Walzengrundkörper 43a vorgesehen sind, deren Winkelerstreckung geringer als diejenige der dazwischen liegenden isolierenden Abschnitte ist. Für bestimmte Meßaufgaben kann eine derartige Konfiguration speziell in Verbindung mit einer Auswertungsschaltung vorteilhaft eingesetzt werden, die eine Auswertung impulsförmiger Meßstromsignale erlaubt. Im übrigen kann eine Querschnittskonfiguration nach Fig. 4B bei spiraligem Verlauf der Umfangsbereiche (ähnlich Fig. 3) in einer Meßanordnung eingesetzt werden, bei der eine gewisse Umschlingung der Meßwalze durch das Meßgut und damit ein ständiger Kontakt beider leitfähiger Bereiche mit dem Meßgut gegeben ist. Bei einer solchen Anordnung kann dann auch mit einer Auswertungsschaltung gearbeitet werden, die auf die Verarbeitung eines kontinuierlichen Meßsignals ausgelegt ist.

Fig. 5 zeigt in einer perspektivischen Darstellung eine weitere Meßwalze 53, bei der auf einem Walzengrundkörper 53a mit einer Drehachse A drei Paare leitfähiger Ringe 53.1/53.2, 53.2/53.4 und 53.5/53.6 mit jeweils einem dazwischen liegenden, schmalen isolierenden Ring 53.8, 53.10 bzw. 53.12 angebracht sind. Zu den Walzenenden hin sind das erste und dritte Meßringpaar durch weitere isolierende Ringe 53.7 und 53.13 isoliert, und zwischen dem ersten und zweiten und zweiten und dritten Meßring-Paar sind breite isolierende Ringe 53.9 bzw. 53.11 vorgesehen. Der Abgriff der Meßsignale erfolgt auch hier in den Endbereichen der Walze durch Kohlebürstenanordnungen 53.14 bzw. 53.15. Ebenso wie die Meßwalze nach Fig. 2 erlaubt die Ausführung nach Fig. 5 die getrennte Erfassung der Restfeuchte einer (hier nicht gezeigten) Textilbahn, auf der die Meßwalze flach aufliegt, in deren Mittenbereich sowie ihren Randbereichen.

Die Ausführung der Erfindung ist nicht auf die oben beschriebenen Beispiele beschränkt, sondern ebenso in einer Vielzahl weiterer Abwandlungen möglich, die im Rahmen fachgemäßen Handelns liegen.

### Bezugszeichenliste

- 1: Feuchtemeßanordnung
- 3; 13; 23; 33; 43; 53: Meßwalze
- 3.1, 3.2; 13.1 bis 13.6; 23.1, 23.2; 33.1 bis 33.4, 43.1, 43.2; 53.1 bis 53.6: leitfähiger Umfangsbereich
- 3.3, 3.4, 13.7, 13.8, 23.3, 23.4: isolierende Stege
- 3.5, 3.6: Messingring
- 5a, 5b: Hilfswalze
- 7: Textilbahn
- 9.1, 9.2; 53.14, 53.15: Kohlebürste
- 13.9, 13.10; 53.7 bis 53.13: isolierende Ringe
- 13a; 43a; 53a: Walzengrundkörper
- 13b: zentrale Bohrung
- 13c: axiale Bohrung
- A: Walzenachse

## Patentansprüche

1. Feuchtemeßanordnung (1) zur Messung des Feuchtegehaltes in einer bewegten Bahn flächigen Materials, insbesondere einer Textilbahn (7), wobei eine insbesondere durch die Materialbahn angetriebene Meßwalze vorgesehen ist, wobei die Meßwalze (3) eine segmentierte Oberfläche mit mindestens zwei durch hochohmige Isolationsabschnitte voneinander getrennten leitfähigen Umfangsbereichen aufweist, welche jeweils mit einem elektrischen Anschluß zur Zuführung bzw. zum Abgriff eines Meßstromes verbunden sind,
**dadurch gekennzeichnet, daß**
die Meßwalze (3) von der Materialbahn (7) unter einem auf deren Segmentierung, insbesondere auf leitfähige Umfangsbereiche (3.1, 3.2) mit zur Walzenachse (A) parallelen Kanten, abgestimmten Umschlingungswinkel berührt wird,
die Meßwalze (3) eine mindestens der Breite der Materialbahn entsprechende Länge hat und mindestens ein Paar von halbzylindrischen leitfähigen Umfangsbereichen (3.1, 3.2) aufweist, die einen Feuchtemeßabschnitt auf der Materialbahn bestimmen.

2. Feuchtemeßanordnung nach Anspruch 1,
**dadurch gekennzeichnet, daß**
die elektrischen Anschlüsse als Schleifringkontakte mit voneinander isolierten leitfähigen Ringen (3.5, 3.6) an mindestens einer der Stirnflächen der Meßwalze und jeweils einem zugeordneten Schleifkontakt (9.1, 9.2), insbesondere einer Kohlebürste, ausgeführt sind.

3. Feuchtemeßanordnung nach einem der vorherigen Ansprüche,
**dadurch gekennzeichnet, daß**
die Breite der Isolationsabschnitte (3.3, 3.4) zur Messung niedriger Restfeuchtegehalte wesentlich kleiner ist als diejenige der leitfähigen Umfangsbereiche (3.1, 3.2).
